# EUROPÄISCHE PATENTANMELDUNG

(11) **EP 0 613 674 A1**
(43) Veröffentlichungstag der Anmeldung: **07.09.1994**
(21) Anmeldenummer: 93103352.6
(22) Anmeldetag: 03.03.1993
(51) Int. Cl.: A61H 39/00, A61N 1/18

(54) **Vorrichtung zur Stimulierung for Akupunkturpunkten**

(71) Anmelder: Hieber, Fritz E.W. Dr. med., D-76530 Baden-Baden (DE)
(72) Erfinder: Hieber, Fritz E.W. Dr. med., D-76530 Baden-Baden (DE)
(74) Vertreter: Trappenberg, Hans

(57) **Zusammenfassung**

Zur Stimulierung wie auch zur Sedierung von Akupunkturpunkten und -linien müssen sowohl die verhältnismäßig schwachen durch Bimetall-Elemente zusammen mit dem Schweiß als Elektrolyt entstehenden Ströme, wie auch die vom Magneten ausgehenden magnetischen Kraftlinien verhältnismäßig lange Zeit einwirken. Die Erfindung gibt eine Vorrichtung an, die eine Dauereinwirkung zuläßt ohne den Patienten zu behindern oder zu entstellen. Die Bimetallelemente (5,6; 8,9) und/oder Magnete können an einem Brillenbügel, einem Stirnband, einem Uhrband (1) oder einem Arm-Schweißband angebracht werden.

## Beschreibung

Die Erfindung betrifft eine Vorrichtung zur Stimulierung oder Sediervng von Akupunkturpunkten und -linien am Unterarm und am Kopf, nämlich insbesondere der Punkte und Linien:
a) 7,8 und 9 des Lungen-Meridians,
b) 4,5,6 und 7 des Meridians für Kreislauf und Sexualität,
c) 4,5,6 und 7 des Herz-Meridians,
d) 4,5,6,7 und 8 des Drei-Erwärmer-Meridians,
e) 5 und 6 des Dickdarm-Meridians, sowie
f) 5,6, und 7 des Dünndarm-Meridians
mittels galvanischer oder magnetischer Beeinflussung.

Die Phänomene der Akupunktur sind, obwohl die Akupunktur schon seit Jahrtausenden in Gebrauch ist, immer noch nicht vollständig geklärt. Bei der klassischen Akupunktur als Methode zur Erkennung und Heilung von Erkrankungen der Atmungs-, Kreislauf- und Verdauungsorgane, des Nervensystems und des Blutes werden silberne und goldene Nadeln an bestimmten, den jeweiligen Organen zugeordneten Hautstellen bis in die Unterhaut eingestochen. Dies soll der Beruhigung, Kräftigung und Wiederherstellung der Funktion des betroffenen Organs dienen. Bei der modernen Akupunktur werden ebenfalls die alt überlieferten Akupunkturpunkte und -linien gereizt beziehungsweise stimuliert, was sowohl durch Einstechen von Nadeln geschehen kann, wie aber auch durch Einwirkung von Druck, Wärme, elektrischem Strom und magnetischen Kraftlinien. Die Erfindung bezieht sich auf die Stimulierung beziehungsweise Sedierung dieser Akupunkturpunkte und -linien durch elektrischen Strom sowie durch magnetische Kraftlinien.

Bei modernen Akupunkturgeräten zur Durchführung dieser Elektroakupunktur wird üblicherweise der Körper mit der einen Elektrode eines Niederspannungs-Stromkreises verbunden. Zum Schließen des Stromkreises dient eine punktförmige Elektrode, die auf dem jeweiligen Akupunkturpunkt aufgesetzt wird. Der Nachteil dieses Verfahrens gegenüber demjenigen beim Einstechen von Nadeln ist darin zu erblicken, daß die Einwirkungen nur kurzzeitig vor sich gehen und daher zum Beeinflussen der Akupunkturpunkte ein verhältnismäßig hoher Strom benötigt wird. Außerdem ist durch diese kurzzeitige Beeinflussung die gewünschte Stimulation, auch bei Anwendung von verhältnismäßig hohen Strömen, nicht gewährleistet.

Die Beeinflussung der Akupunkturpunkte beziehungsweise Akupunkturlinien durch magnetische Kraftlinien kann nur durch langdauernde Einwirkung erfolgen. Kurzzeitige Einwirkung magnetischer Kraftlinien mit unter einer Stunde liegenden Einwirkungszeiten sind zwar auch aus der Medizin bekannt, dienen jedoch anderen Zwecken, insbesondere der Heilung von Knochenbrüchen.

Aufgabe der Erfindung ist es mithin eine Vorrichtung anzugeben, die es gestattet, die Stimulierung beziehungsweise Sedierung der Akupunkturpunkte beziehungsweise -linien über einen längeren Zeitraum durchzuführen, ohne daß hierdurch der Patient behindert oder auch durch sichtbare äußere Einrichtungen entstellt wird. Für die Stimulierung beziehungsweise Sedierung der eingangs aufgeführten Akupunkturpunkte und -linien wird hierzu, nach der Erfindung, sowohl ein um den Unterarm beim Handgelenk herumgeschlungenes Band vorgeschlagen, an dem, mit der freien Seite zum Unterarmgewebe zeigend, Bimetall-Elemente und/oder Magnete angebracht sind, wie auch eine Anordnung dieser Bimetallelemente und/oder Magnete an einem Brillenbügel oder an einem Stirnband, wobei diese Elemente sowohl zum Kopf wie auch zur Ohrmuschel-Rückseite weisen können. Vorteilhafterweise wird als Band ein Uhrband verwendet, das keinerlei Rückschlüsse auf den Therapiezweck erkennen läßt. Dem gleichen Zweck dienlich sind auch am Unterarm, beim Handgelenk, anzulegende Schweißbänder.

Als "Bimetall-Elemente" werden gemäß der Erfindung Elemente bezeichnet, die zumindest auf der, auf das Gewebe weisenden Seite aus zwei Metallen zusammengefügt sind, die zusammen mit dem dort entstehenden Schweiß als Elektrolyt ein galvanisches Element bilden, bei dem Strom zwischen diesen beiden Metallen fließt, der sich über die dort befindlichen Akupunkturpunkte und -linien ausgleicht. Als Metalle werden zweckmäßigerweise Paarungen vorgesehen, die in der Spannungsreihe der chemischen Elemente möglichst weit auseinanderliegen, wie beispielsweise Gold/Stahl oder Gold/Aluminium. Selbstverständlich ist auch eine Paarung Gold/Kupfer möglich, um gleichzeitig mit der galvanischen Beeinflussung auch ein erwünschtes Eindringen von Kupferionen in das Gewebe zu erreichen.

Sowohl die Bimetall-Elemente wie auch die Magnete werden zweckmäßigerweise auf das Band beziehungsweise den Brillenbügel umfassenden verschieb- und feststellbaren Schiebern angebracht oder auch in das Gewebe des Stirnbandes oder des Arm-Schweißbandes eingefügt, um eine möglichst genaue Justierung dieser beeinflussenden Elemente auf an der jeweiligen Körperfläche lokalisierten Akupunkturpunkte und -linien zu ermöglichen. Hinsichtlich dieser Justierung ist bei der erfindungsgemäßen Vorrichtung noch darauf hinzuweisen, daß durch die stete Bewegung des Bandes beim Tragen, wie auch des Brillenbügels, immer wieder unterschiedliche Punkte durch die aufgebrachten Bimetall-Elemente beziehungsweise Magnete angesprochen werden beziehungsweise eine durch die Bewegungen hervorgerufene pulsierende Beeinflussung der dort befindlichen Akupunkturpunkte und -linien erreicht wird.

Schließlich kann bei der Verwendung eines Uhrbandes auch noch der Uhrboden einer solchen Armbanduhr als Bimetall-Element benutzt werden dadurch, daS beispielsweise der Boden einer solchen Uhr aus Stahl gefertigt ist und der umgebende Rand des Gehäuses aus Gold besteht oder zumindest eine Goldauflage aufweist.

Auf der Zeichnung ist ein Ausführungsbeispiel des Erfindungsgegenstandes anhand eines Uhrbandes schematisch dargestellt und zwar zeigen:

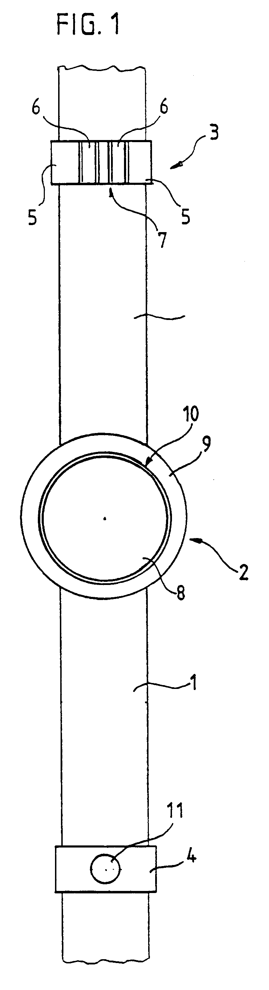

Fig. 1 die Unterseite eines Uhrbandes,
Fig. 2 und
Fig. 3 die Seitenansicht von Schiebern und
Fig. 4 die Seitenansicht der Uhr-Unterseite.

Ein Uhrband (1) mit Uhr (2) ist von zwei Schiebern (3,4) umschloßen, die auf dem Uhrband (1) festgelegt sind. Der Schieber (3) weist an seiner Unterseite Zonen verschiedener Metalle, in diesem Falle Zonen aus Stahl (5) und aus Gold (6) auf. Die Zonen (5,6) sind jeweils durch Nuten (7) voneinander getrennt. Zusammen mit dem auf der Haut des Unterarmgewebes entstehenden schweiß bilden sich so durch die verschiedenen Metalle (5,6) galvanische Elemente, deren Strom zwischen diesen Zonen (5,6) durch das Unterarmgewebe hindurchfließt und somit die dort befindlichen Akupunkturpunkte und -Linien stimuliert beziehungsweise sediert. Die gleiche Wirkung zeigt sich bei den Akupunkturpunkten und -Linien, an denen die Unterseite der Uhr (2) aufliegt. Diese Unterseite bildet ebenfalls ein galvanisches Element, bestehend aus dem Stahlboden (8) und umgebendem Goldrand (9) des Uhrgehäuses. Auch hier sind wiederum die beiden Metalle durch eine kreisringförmige Nut (10) zur Tiefeneinwirkung der galvanischen Ströme in das Unterarmgewebe voneinander getrennt.

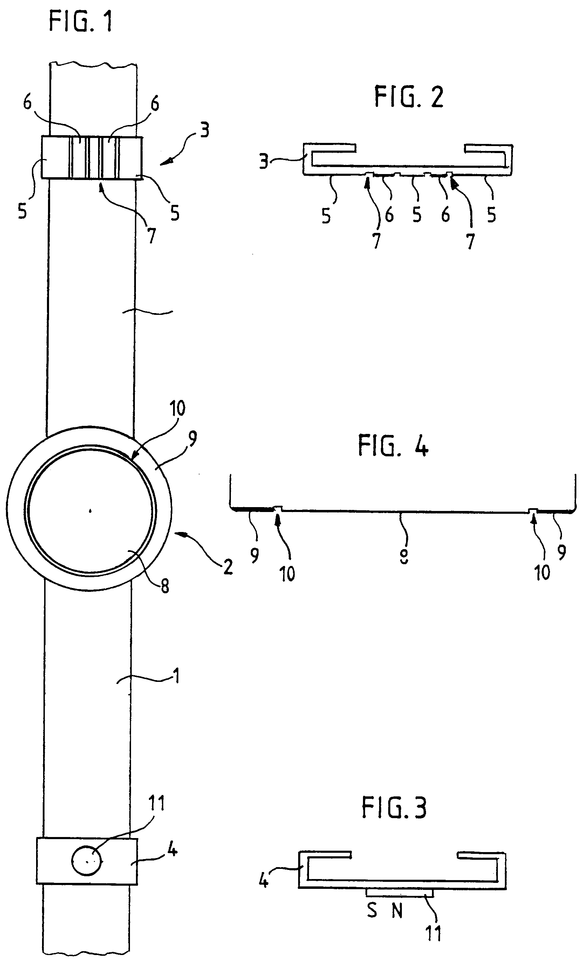

Fig. 3 schließlich zeigt die Seitenansicht eines Schiebers (4), der mit einem Magneten (11) bestückt ist, dessen Kraftlinien zwischen Nord- (N) und Südpol (S) durch das Unterarmgewebe hindurch verlaufen.

## Patentansprüche

1. Vorrichtung zur Stimulierung oder Sedierung von Akupunkturpunkten und -linien am Unterarm und am Kopf, nämlich insbesondere Punkte und Linien von
a) 7,8 und 9 des Lungen-Meridians,
b) 4,5,6 und 7 des Meridians für Kreislauf und Sexualität,
c) 4,5,6 und 7 des Herz-Meridians,
d) 4,5,6,7 und 8 des Drei-Erwärmer-Meridians,
e) 5 und 6 des Dickdarm-Meridians, sowie
f) 5,6 und 7 des Dünndarm-Meridians
mittels galvanischer oder magnetischer Beeinflussung,
gekennzeichnet
durch ein um den Unterarm beim Handgelenk herumzuschlingendes Band, an dem mit der freien Seite zum Unterarmgewebe zeigend, Bimetall-Elemente (5,6,8,9) und/oder Magnete (11) angebracht sind, wie auch eine Anordnung dieser Bimetallelemente (5,6,8,9) und/oder Magnete (11) an einem Brillenbügel oder an einem Stirnband, wobei diese Elemente (5,6,8,9,11) sowohl zum Kopf wie auch zur Ohrmuschel-Rückseite weisen können.

2. Vorrichtung nach Anspruch 1,
dadurch gekennzeichnet,
daß das Band (1) ein Uhrband ist.

3. Vorrichtung nach Anspruch 1,
dadurch gekennzeichnet,
daß das Band ein Arm-Schweißband ist.

4. Vorrichtung nach Anspruch 1 oder 2,
dadurch gekennzeichnet,
daß die Bimetall-Elemente (5,6) und/oder Magnete (11) auf das Band (1) / den Brillenbügel umfassende verschieb- und feststellbaren Schiebern (3,4) angebracht sind.

5. Vorrichtung nach Anspruch 1,
dadurch gekennzeichnet,
daß die Bimetall-Elemente (5,6) insbesondere die Paarung Gold/Aluminium, Gold/Stahl oder Gold/Kupfer aufweisen.

6. Vorrichtung nach Anspruch 2,
dadurch gekennzeichnet,
daß der Boden (8) der Uhr (2) an dem das Uhrband (1) befestigt ist, als Bimetall-Element mit vorzugsweise der Paarung Gold/Stahl ausgebildet ist.
